# EUROPEAN PATENT SPECIFICATION

(11) **EP 4 487 958 B1**
(45) Date of publication and mention of the grant of the patent: **05.11.2025**
(21) Application number: 23183188.4
(22) Date of filing: 04.07.2023
(51) Int. Cl.: B01L 3/08, C12M 1/06, C12M 3/04, C12M 1/00

(54) **INLAY FOR A FLASK AND METHOD FOR INSERTING AN INLAY INTO A FLASK**
EINLAGE FÜR EINE FLASCHE UND VERFAHREN ZUM EINSETZEN EINER EINLAGE IN EINE FLASCHE
INSERT POUR FLACON ET PROCÉDÉ D'INSERTION D'UN INSERT DANS UN FLACON

(43) Date of publication of application: 08.01.2025
(73) Proprietor: Hochschule für angewandte Wissenschaften München in Vertretung des Freistaates Bayern, 80335 München (DE)
(72) Inventor: Hanisch, Patrick, 85241 Hebertshausen (DE); Huber, Robert, 83080 Oberaudorf (DE)
(74) Representative: Lucke, Andreas

(56) References cited:
- GB-A- 1 243 750
- JP-B2- 2 853 950
- US-A- 4 665 035

## Description

### TECHNICAL FIELD

The disclosure relates to an inlay for a flask, such as a shake flask for cultivating microorganisms, in particular to improve the oxygen transfer into a liquid in the flask, and/or with improved sensoric access to the liquid, for example, to determine the oxygen content of the liquid.

### BACKGROUND

Microorganisms are cultivated in different apparatus, adapted to the specific needs of the respective microorganism. Aerobic microorganisms require oxygen. In cultivating apparatus typically used at the lab scale, the oxygen is provided into a solution with the microorganism, by keeping the solution in a flask, and shaking the flask with a permeable closure for ambient air, e.g. a cotton plug. The most commonly used flasks in this context are Erlenmeyer flasks, such as narrow-neck Erlenmeyer flasks according to ISO 1773 or wide-neck Erlenmeyer flasks according to ISO 24450. However, the oxygen transfer into the solution is limited in this setup. Typically, the oxygen content of the solution is high at its interface to the air, but the transfer of oxygen from this oxygen rich layer to the rest of the solution is slow and inefficient. Furthermore, the ratio of surface area to culture volume as well as the shaking speed and shaking diameter play a crucial role for oxygen transfer. To improve the oxygen transfer, baffled flasks have been developed. A baffled flask has a shape similar to the one of a corresponding Erlenmeyer flask, but with baffles at its bottom inner surface or at the side wall inner surface. When the baffled flask is shaken, the baffles increase mixing within the solution and hence improve the oxygen transfer from the oxygen rich layer to the rest of the solution. However, the shape of the baffles can vary due to the production process, in particular, for flasks made of glass, and so varies the oxygen transfer rate, resulting in undesirable variations when cultivating microorganisms. Moreover, the production of baffled flasks is technically more complicated than the production of a corresponding Erlenmeyer flask (without baffles), and consequently their price is high, ranging from about 5 to 10 times the price of the corresponding Erlenmeyer flask.

JP 2853950 B2 and US 4,665,035 A describe conventional flasks with baffles. GB 1 243 750 A discloses a test container for the cultivation and inspection of micro-organisms.

### OVERVIEW

In view of the technical problems laid out above, there is a need for improvements related to flasks, such as flasks applied for cultivating microorganisms. This objective is achieved with an inlay for a flask according to claim 1, and with a method for inserting an inlay into a flask according to claim 13. The dependent claims refer to advantageous embodiments.

According to a first aspect, an inlay for a flask comprises a bottom portion, a side portion, an intermediate portion, and a baffle element. The bottom portion comprises a downward-facing or downward-and-outward-facing first surface for abutting a bottom inner surface of the flask. The side portion comprises an outer surface for abutting a side inner surface of the flask, wherein the outer surface faces outwards with respect to a vertical reference line intersecting the inlay. The intermediate portion mechanically connects the bottom portion and the side portion. The baffle element extends away from the bottom portion, from the side portion, and/or from the intermediate portion for extending into an inner volume of the flask. An inner angle between the first surface and the outer surface is at least 50°, wherein the inner angle refers to an angle on the side of the vertical reference line in a vertical cross section through the inlay. The inlay comprises a first flexible material adapted to allow for bending said side portion inwards. The inlay is adapted to allow for folding the inlay with respect to a horizontal crease line to insert the inlay into the flask. The inlay comprises, along a vertical plane comprising the horizontal crease line, a second flexible material to allow for said folding.

The respective inlay provides baffle elements (which may alternatively referred to as stirring elements) to a flask, such as an Erlenmeyer flask, as for example, also a baffled flask does. Consequently, the inlay improves the mixing within the flask, and thus the oxygen transfer rate into a solution contained in the flask when the flask is shaken.

Compared to the baffled flask, the inlay can be fabricated much more easily, for example by molding, and, consequently, its price is much lower.

The accuracy of the produced (e.g., by molding) baffle elements is much higher than the accuracy in the production of baffled flasks, in particular, in the production of baffled flasks made from glass. This improves the reproducibility of the microorganism culture.

Various inlays with different sizes, inner angles, or baffle elements, can easily and economically be provided, such that a user may economically keep a stock of respective inlays and select the best one for the needs of his current microorganism culture.

The inlay can be removed from the flask when no longer needed, and both the flask and the inlay can be reused, for example, in another combination of flask and inlay.

According to an alternative embodiment, an inlay for a flask comprises a bottom portion, a side portion, an intermediate portion, and a baffle element. The bottom portion comprises a downward-facing bottom surface for providing a force-fit connection to a bottom inner surface of the flask. The side portion comprises an outer surface for providing a force-fit connection to a side inner surface of the flask. The outer surface faces outwards with respect to a vertical reference line intersecting the inlay. The intermediate portion mechanically connects the bottom portion and the side portion. The baffle element extends away from the bottom portion, from the side portion, and/or from the intermediate portion for extending into an inner volume of the flask. The inlay comprises a first elastomer for providing the force-fit connection of the downward-facing bottom surface to the bottom inner surface of the flask and the force-fit connection of the outer surface to the side inner surface of the flask. An inner angle between the bottom surface and the outer surface is at least 50°, wherein the inner angle refers to an angle on the side of the vertical reference line in a vertical cross section through the inlay. The first elastomer allows for bending said side portion inwards against an elastic restoration force, said restoration force promoting said force-fit connection. The inlay is adapted to allow for folding the inlay with respect to a horizontal crease line. The inlay comprises, along a vertical plane comprising the horizontal crease line, a second elastomer, wherein the second elastomer is adapted to allow for said folding, and is adapted to unfold the inlay upon said folding, for inserting the inlay into the flask.

In some embodiments, a ratio of a height of the inlay along the vertical direction and a maximum width of the inlay in a horizontal direction is at most 0.5, or at most 0.4, or at most one third.

A height of the inlay may be at most 10 cm, or at most 6 cm, or at most 5 cm, or at most 4 cm.

This facilitates easy and reliable folding to a sufficiently small size to insert the inlay into the flask.

A height of the inlay may be at least 0.5 cm, or at least 0.75 cm, or at least 1 cm.

Material compositions of the first flexible material and the second flexible material may be identical.

The inlay may be an integral part, such as an integral part formed by molding and/or an integral part composed of a flexible material providing the first flexible material and/or the second flexible material.

Corresponding embodiments facilitate mass production, as the inlay may be produced in large numbers by molding, in particular in a single molding step.

A surface of the inlay may be hydrophilic.

Alternatively, or in addition, a surface of the bottom portion and/or of the side portion and/or of the intermediate portion and/or of the baffle element and/or of the first flexible material and/or of the second flexible material may be hydrophilic.

The hydrophilic surface(s) improves biocompatibility and also improves the wettability of the inlay, hence improving the oxygen transfer further.

The intermediate portion may be arranged between the bottom portion and the side portion.

The intermediate portion or an outward-facing surface of the intermediate portion may extend from the outer surface to the first surface, in particular, from a bottom edge of the outer surface and/or to an outer edge of the first surface.

In some embodiments, the outward-facing surface of the intermediate portion comprises an orientation with a downward component and/or with an outward component.

Alternatively, or in addition, the outward-facing surface of the intermediate portion may comprise a convex shape, in particular, with a radius in a range from 6 mm to 6 cm.

In corresponding embodiments, the intermediate portion and/or its outward-facing surface is adapted to abut the flask at the transition between its bottom inner surface and its side inner surface, ideally flush.

The first flexible material may be a first elastomer.

According to some embodiments, the first elastomer is adapted to promote a force-fit connection of the first surface to the bottom inner surface of the flask and/or to promote a force-fit connection of the outer surface to the side inner surface of the flask.

According to some embodiments, the first elastomer is adapted to, upon the bending the side portion inwards, provide an elastic restoration force against the bending, said restoration force promoting said force-fit connection of the side portion to the side inner surface of the flask and/or of the first surface to the bottom inner surface of the flask.

The second flexible material may be a second elastomer.

According to some embodiments, the second elastomer is adapted to allow for said folding, and is adapted to unfold the inlay upon said folding.

In corresponding embodiments, the inlay is securely held in place using the force-fit connection(s) provided by the elastomer(s). This connection is detachable, so that the inlay and the flask may be separated from each other after usage together, and may be reused separately.

The downward-facing or downward-and-outward-facing first surface may be a downward-facing bottom surface of the bottom portion.

According to some embodiments, in a horizontal plane intersecting the bottom portion and/or the first surface and/or the intermediate portion, a shape of the bottom portion and/or a shape of the first surface and/or a shape of the intermediate portion is a ring segment or a ring.

According to some embodiments, in a horizontal plane intersecting the bottom portion and/or the first surface, a shape of the bottom portion and/or a shape of the first surface is a ring segment or a ring. A width of the ring segment or of the ring may be at least 2 millimeters, or at least 4 mm, or at least 6 mm.

The bottom portion and/or the first surface may comprise an opening, in particular, comprising its center/their centers or centered in its center/their centers.

The opening may have a width or a diameter of at least 1 cm or of at least 2 cm or of at least 3 cm.

The opening may have a circular shape and/or the opening may be centered in the first surface.

In corresponding embodiments, the ring-shape or the opening facilitates inserting the inlay into the flask, and improves the flush abutting of the downward-facing or downward-and-outward-facing first surface and the bottom inner surface of the flask.

The inlay may be an integral part composed of an elastomer material providing the first elastomer and the second elastomer.

The first elastomer and/or the second elastomer may comprise or may be rubber or silicone.

Material compositions of the first elastomer and the second elastomer may be identical.

The intermediate portion and/or the side portion may comprise or may be composed of the second elastomer.

The bottom portion may comprise or may be composed of the first elastomer.

The side portion and/or the intermediate portion may comprise or may be composed of the first elastomer.

The baffle element may comprise or may be composed of the first elastomer.

A thickness of the bottom portion, in particular along the vertical direction, may be at most 6 mm, or at most 5 mm, or at most 4 mm.

A thickness of the side portion, in particular along a radial direction with respect to the vertical reference line, may be at most 6 mm, or at most 5 mm, or at most 4 mm.

A thickness of the intermediate portion may be at most 6 mm, or at most 5 mm, or at most 4 mm.

Respective thicknesses ensure that the material is indeed flexible, for example when an elastomer material or a suitable polymer material is used.

The first surface may be flat and/or may extend in a horizontal plane.

The inner angle may be at least 60°, or at least 70°, or at least 75°, or at least 80°, or at least 85°, or at least 90°, or more than 90°, or at least 91°, or more than 95°.

A height of the outer surface along the vertical direction may be at least 2 mm, or at least 4 mm, or at least 6 mm.

A width or a diameter of the inlay in the horizontal direction may be in a range from 4 cm to 30 cm.

The vertical reference line may extend through a center of the inlay and/or of the bottom portion and/or of the side portion and/or of the intermediate portion.

The horizontal crease line may extend through the bottom portion and/or through the intermediate portion and/or through the first surface, in particular, through a center thereof.

The vertical cross section and/or the vertical plane may comprise the vertical reference line.

According to some embodiments, the outer surface or at least a section of the outer surface extends along a side of a reference cylinder, wherein an axis of the reference cylinder is aligned with the vertical reference line, and wherein, optionally, the first surface and/or the bottom portion and/or the intermediate portion and/or the baffle element is/are fully comprised in the reference cylinder.

According to some embodiments, the bending the side portion inwards bends the side portion towards the vertical reference line and/or reduces the inner angle, wherein, optionally, the elastic restoration force acts to bend the side portion away from the vertical reference line and/or to increase the inner angle.

The baffle element may comprise or be composed of an elastomer.

The baffle element may extend upwards from the bottom portion.

The baffle element may extend inwards from the side portion.

The baffle element may be recessed above the horizontal crease line.

The inlay may further comprise a second opening adapted for receiving an optode or for permitting a line of sight from below the flask to the inner volume of the flask, in particular, wherein the second opening has an extrusion shape corresponding to an extrusion of a base in a horizontal plane along the vertical direction.

The inlay may further comprise a recess adapted to receive a feeding pill or a feeding cartridge or a buffer pill or a buffer cartridge, in particular, wherein the recess faces upwards or inwards, such as from the bottom portion and/or from the intermediate portion.

The inlay may further comprise a light-emitting diode mounted to the inlay, in particular, to the bottom portion and/or to the intermediate portion.

The light-emitting diode is particularly beneficial for cultivating phototrophic organisms such as algae.

The flask may be an Erlenmeyer flask, in particular a wide-neck or a narrow-neck Erlenmeyer flask, such as a narrow-neck Erlenmeyer flask according to ISO 1773 or a wide-neck Erlenmeyer flask according to ISO 24450 or an Erlenmeyer flask according to DIN 4797.

According to a second aspect, a method is provided for inserting an inlay into a flask. The inlay comprises a second flexible material and a baffle element. The method comprises folding the second flexible material with respect to a crease line; aligning the crease line parallel to an axis of the flask; inserting the folded inlay into the flask with the crease line aligned with the axis of the flask; and unfolding, unfolding the second flexible material, the inlay in the flask, such that a downward-facing or downward-and-outward-facing first surface and/or an outer surface of the inlay abuts an inner surface of the flask, and that the baffle element extends into an inner volume of the flask.

The second flexible material may be a second elastomer. The second elastomer may promote the unfolding the inlay in the flask.

The inlay may be unfolded by the second elastomer such that the crease line is orientated parallel to a bottom surface of the flask and/or orthogonal to the axis of the flask.

According to some embodiments, the inlay is unfolded such that the inlay is fully positioned below a neck portion of the flask.

The method may further comprise fixing the inlay in the flask and/or removing a section of the inlay in the flask, such as by etching or ashing.

According to some embodiments, the inlay comprises a bottom portion comprising the first surface, a side portion comprising the outer surface, an intermediate portion mechanically connecting the bottom portion and the side portion, and a first flexible material, such as a first elastomer, and the unfolding comprises: arranging the first surface to abut a bottom inner surface of the flask; and arranging the outer surface to abut a side inner surface of the flask.

In embodiments with the first elastomer, the method may further comprise providing, by the first elastomer, a force-fit connection of the first surface to the bottom inner surface of the flask and/or a force-fit connection of the outer surface to the side inner surface of the flask.

### BRIEF DESCRIPTION OF THE FIGURES

The techniques of the present disclosure and the advantages associated therewith will be best apparent from a description of exemplary embodiments in accordance with the accompanying drawings, in which:
- Fig. 1a: gives a top view of an inlay according to a first embodiment;
- Fig. 1b: gives a sectional view of the inlay according to the first embodiment;
- Fig. 2a: gives a sectional view of an inlay according to another embodiment;
- Fig. 2b: gives a sectional view of an inlay according to another embodiment;
- Fig. 3a: gives sectional views of inlays according to other embodiments;
- Fig. 3b: gives sectional views of inlays according to other embodiments;
- Fig. 3c: gives a sectional view of an inlay according to another embodiment;
- Fig. 3d: gives a sectional view of an inlay according to another embodiment;
- Fig. 4a: gives a perspective view of an inlay according to another embodiment;
- Fig. 4b: gives another perspective view of the inlay according to the embodiment of Fig. 4a;
- Fig. 4c: gives a side view of the inlay according to the embodiment of Fig. 4a, Fig. 4b;
- Fig. 5a: illustrates a method for inserting an inlay into a flask according to an embodiment;
- Fig. 5b: further illustrates the method for inserting the inlay into the flask;
- Fig. 5c: further illustrates the method for inserting the inlay into the flask;
- Fig. 5d: further illustrates the method for inserting the inlay into the flask;
- Fig. 6: illustrates a method for inserting an inlay into a flask according to another embodiment;
- Fig. 7a: gives a perspective view of an inlay according to an embodiment with a baffle element extending upwards from a bottom portion;
- Fig. 7b: gives a perspective view of an inlay according to another embodiment with a baffle element extending upwards from a bottom portion;
- Fig. 7c: gives a perspective view of an inlay according to another embodiment with a baffle element extending upwards from a bottom portion;
- Fig. 7d: gives a perspective view of an inlay according to another embodiment with a baffle element extending upwards from a bottom portion;
- Fig. 7e: gives a perspective view of an inlay according to another embodiment with a baffle element extending upwards from a bottom portion;
- Fig. 8a: gives a perspective view of an inlay according to an embodiment with a baffle element extending inwards from a side portion;
- Fig. 8b: gives a perspective view of an inlay according to another embodiment with a baffle element extending inwards from a side portion;
- Fig. 8c: gives another perspective view of the embodiment of Fig. 8b;
- Fig. 8d: gives a perspective view of an inlay according to another embodiment with a baffle element extending inwards from a side portion;
- Fig. 8e: gives another perspective view of the embodiment of Fig. 8d;
- Fig. 8f: gives a cross-sectional view of the embodiment of Fig. 8d and Fig. 8e;
- Fig. 9a: gives a perspective view of a body with second openings;
- Fig. 9b: gives a perspective view of a body with second openings and recesses;
- Fig. 9c: gives a perspective view of a body with light-emitting diodes; and
- Fig. 10: illustrates oxygen transfer rates.

### DETAILED DESCRIPTION OF EMBODIMENTS

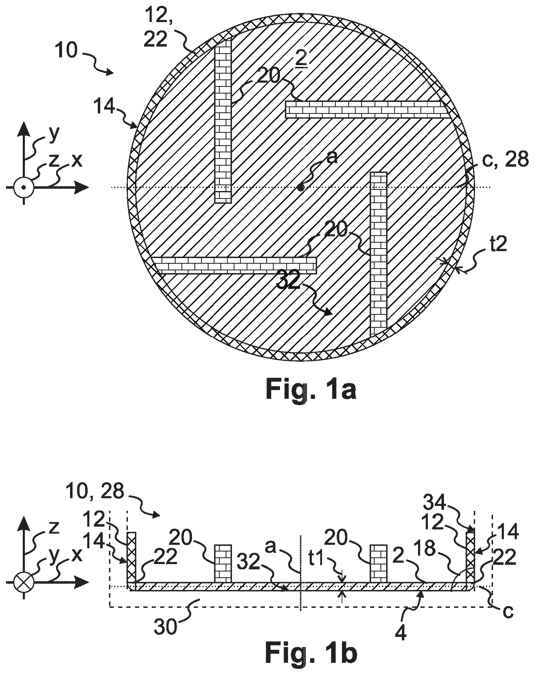

Fig. 1a, Fig. 1b show an inlay 10 according to a first embodiment. Fig. 1a gives a top view, and Fig. 1b gives a cross-sectional view along the vertical x, z plane 28 comprising the line c in Fig. 1a. In Fig. 1b, a bottom portion of a flask 30 (not shown in Fig. 1a) is indicated by dashed lines.

The inlay 10 comprises a bottom portion 2 and a side portion 12. An intermediate portion 22, which, in the depicted embodiment, is the edge between the bottom portion 2 and the side portion 12, and connects the portions 2, 12.

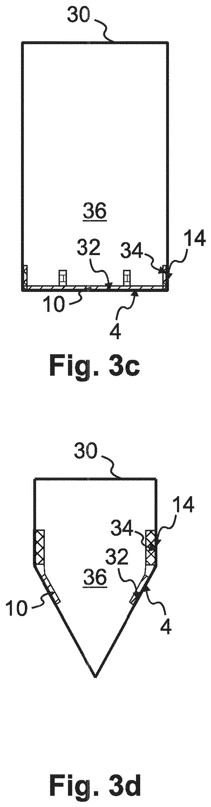

The bottom portion 2 has a first surface 4, adapted to abut a bottom inner surface 32 of a flask 30 (not shown in Figs. 1a). In the depicted embodiment, the first surface 4 faces downwards, but in other embodiments, it is a downward-and-outward-facing surface 4 (see Fig. 3d).

The side portion 12 has an outer surface 14, which is adapted to abut a side inner surface 34 of the flask 30.

The inner angle 18 between the first surface 4 and the outer surface 14 is selected according to the shape of a flask 30, that the inlay 10 is to be inserted into, and amounts to at least 50°, and preferably at least 70° to implement an inlay 10 for an Erlenmeyer flask.

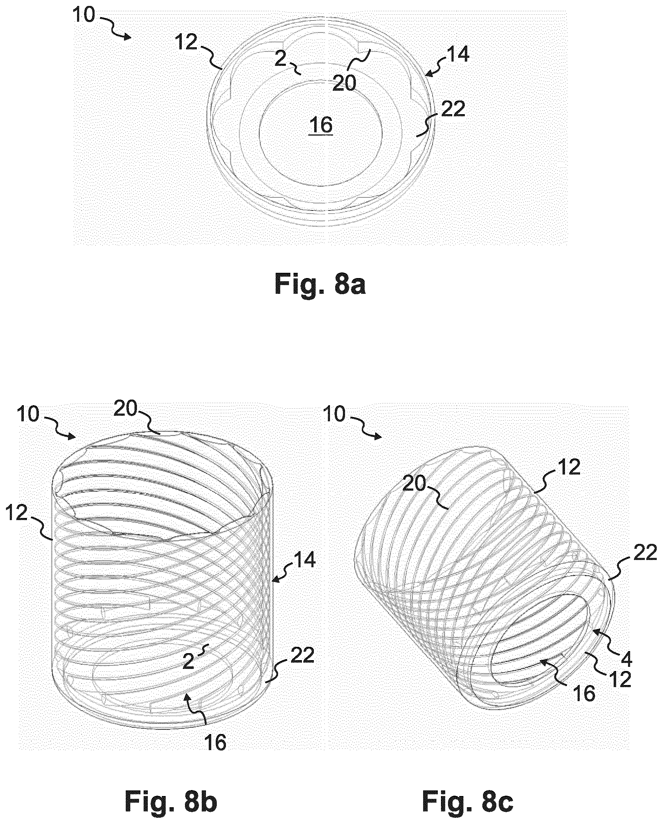

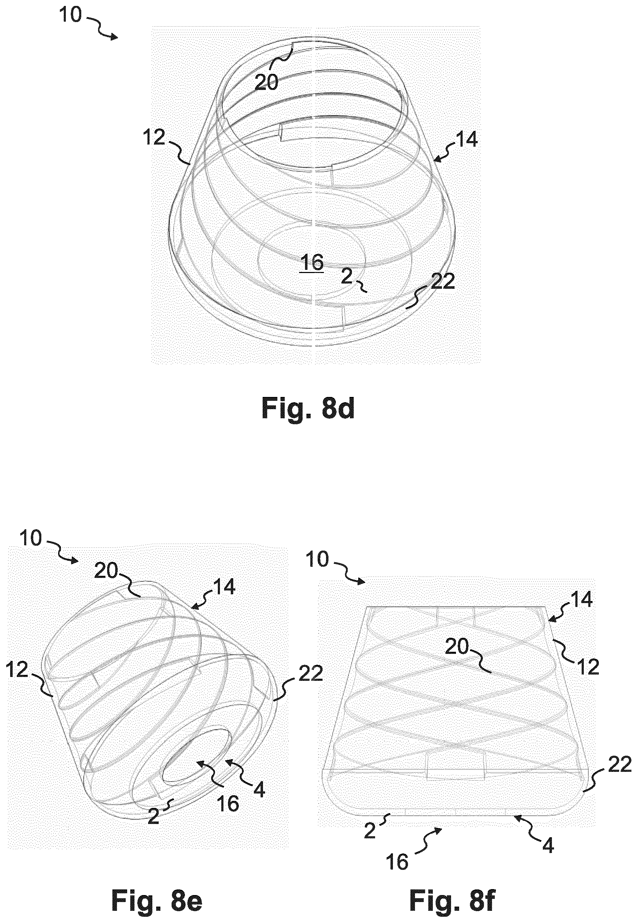

Baffle elements 20 (stirring elements 20) in the form of fins 20 extend upwards from the bottom portion 2. In alternative embodiments, see Fig. 8a, Fig. 8b, Fig. 8c, Fig. 8d, Fig. 8e, the baffle elements 20 extend inwards from the side portion 12.

Where the vertical plane 28 intersects the inlay 10, the inlay comprises a (first) flexible material. Consequently, the inlay 10 can be folded along the line c. In this sense, line c serves as a crease line.

The side portion 12 of the inlay 10 can be bent inwards towards the axis a. For this purpose, the bottom portion 2 in the vicinity of the intermediate portion 22, the intermediate portion 22, and/or the side portion 12 comprises a flexible material.

In the embodiment depicted in Fig. 1a, Fig. 1b, the outer contour of the bottom portion 2 as well as the outer contour of the side portion 12 is a full circle, i.e., in the top view of Fig. 1a. In alternative embodiments (not shown), this full circle is interrupted. For example, the height of the side portion 12 and/or of the intermediate portion 22 and/or of the baffle element(s) 20 may be reduced above the crease line c or the respective element may be absent above the crease line c to facilitate easier folding with respect to the crease line c. In alternative embodiments (not shown), the outer contour of the bottom portion 2 as well as the outer contour of the side portion 12 is a square or a rectangle, i.e., in a top view.

Thicknesses t1, t2 of the bottom and the side portions 2, 12, respectively, are preferably no more than 6 mm, 5 mm, or 4 mm, to ensure that the material of the respective portion is flexible.

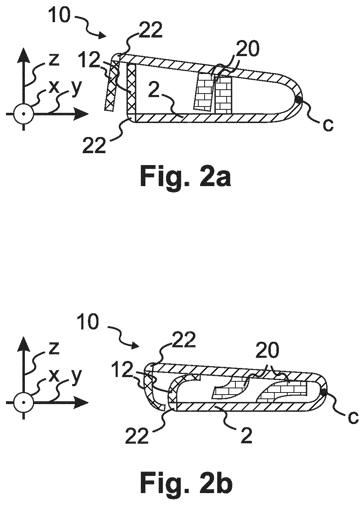

Fig. 2a, Fig. 2b illustrate the folding of the inlay 10, giving cross sectional views of folded inlays according to different embodiments.

The inlay 10 of Fig. 2a comprises rigid baffle elements 20. The rigid baffle elements 20 may be advantageous in some embodiments to ensure a high oxygen transfer rate.

The inlay 10 of Fig. 2b comprises flexible baffle elements 20, i.e., baffle elements 20 comprising a flexible material. Advantageously, in this embodiment, the inlay 10 can be folded to a smaller height (as compared to the inlay 10 of the embodiment depicted in Fig. 2a). This makes inserting the inlay 10 into a flask easier, faster, and more reliable.

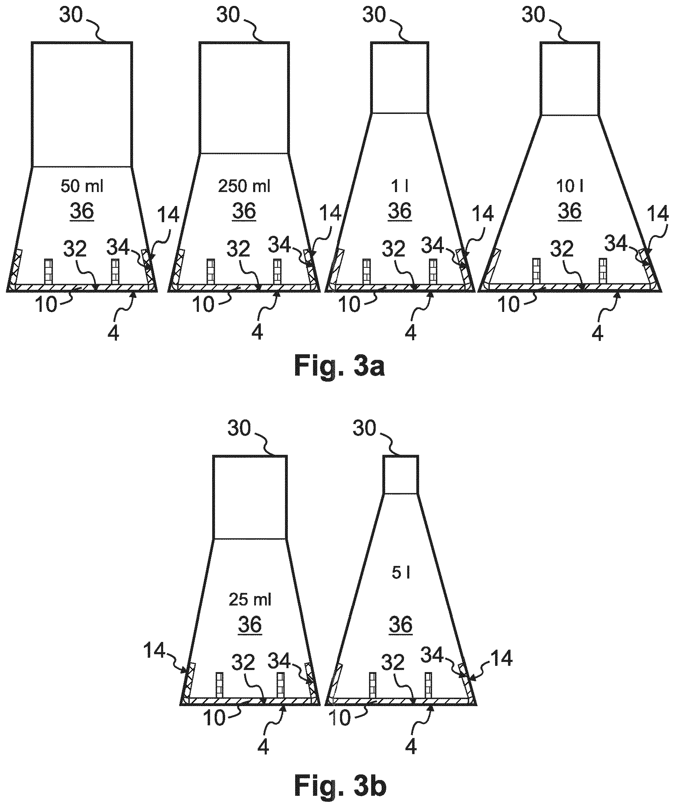

Fig. 3a, Fig. 3b, Fig. 3c, Fig. 3d illustrate various embodiments of the inlay 10 for different flasks 30.

Before going into the details of the individual embodiments, common properties of all the depicted embodiments will be described. These do not only apply to the embodiments of Fig. 3a, Fig. 3b, Fig. 3c, Fig. 3d, but as well to the other embodiments of this description.

In some embodiments, the inlay 10 is fixed in the flask 30, i.e., using a fixing element such as a glue or a resin. In corresponding embodiments, the first and/or second flexible material can be selected from a wide range of materials, including plastics, for as long as they are sufficiently thin to be flexible and allow for a bending with respect to the crease line c (see Fig. 2a, Fig. 2b) by more than 90°. The first and/or second flexible material can as well be an elastomer, such as silicone or rubber.

However, preferably, the inlay 10 is adapted to be held in place in the flask 10 using force-fit connections, i.e., a force-fit connection of the first surface 4 to the bottom inner surface 32 of the flask and/or a force-fit connection of the outer surface 14 to the side inner surface 34 of the flask 30. For this purpose, the first and/or second flexible material is/are an elastomer material.

The second elastomer material, along the crease line c, provides a restoration force against the folding of the inlay 10 with respect to the crease line c. In other words, when the force which caused the folding is released, the inlay 10 is unfolded by the second elastomer, or by the restoration force it provides, respectively. This pushes the outer surface 14 against the side inner surface 34 of the flask 30, and the first surface 4 against the bottom inner surface 32 of the flask 30, promoting said force-fit connections.

The first elastomer material, comprised in the side portion 12, the intermediate portion 22, and/or the bottom portion 2 in the vicinity of the intermediate portion 22, provides an elastic restoration force against the bending inwards of the side portion 12. Consequently, this restoration force pushes the outer surface 14 against the side inner surface 34 of the flask 30, and the first surface 4 against the bottom inner surface 32 of the flask, further promoting said force-fit connections.

The advantage of the embodiments adapted to provide the force-fit connection(s) over the ones fixed in the flask 30 is rooted in the force-fit connection(s) being detachable. Consequently, the inlay 10 can be removed from the flask 30 without damaging or destroying it, and can be reused after cleaning or in another flask 30.

The first and/or second elastomer material is/are typically rubber or silicone. Those also offer the advantage that the flask 30 can be autoclaved with the inlay 10 inside.

Inlays 10 for being fixed to the flask 30 have inner angles 18 corresponding to the inner angle between the bottom inner wall 32 and the side inner wall 34 of the respective flask 30.

Inlays 10 for force-fit connection(s) to the flask 30 have inner angles 18 slightly larger than the inner angle between the bottom inner wall 32 and the side inner wall 34 of the respective flask 30. A difference of at least 1° is preferred, for example a difference of 1° or 2° has proven sufficient.

Fig. 3a illustrates inlays 10 for wide-neck Erlenmeyer flasks 30 according to ISO 24450. The inner angles between the bottom inner walls 32 and the side inner walls 34 of those Erlenmeyer flasks 30 are in the range from 71 - 79°, with generally larger angles for smaller flasks. The inlay 10 for the wide-neck Erlenmeyer flasks 30 has an inner angle 18 of at least 70°.

Fig. 3b illustrates inlays 10 for narrow-neck Erlenmeyer flasks 30 according to ISO 1773. The inner angles between the bottom inner walls 32 and the side inner walls 34 of those Erlenmeyer flasks 30 are in the range from 75 - 79°, with generally larger angles for smaller flasks. The inlay 10 for the narrow -neck Erlenmeyer flasks 30 has an inner angle 18 of at least 75°.

Fig. 3c illustrates an inlay 10 for a flask 30 with vertical inner side walls, i.e., with an inner angle 18 of 90° between the bottom inner wall 32 and the side inner walls 34 of the flask 30.

Fig. 3d illustrates an inlay 10 for a flask 30 typically used with a centrifuge (not shown). This type of flask 30 has an inner angle 18 in the range from 95° to 120° between its bottom inner wall 32 and its side inner walls 34.

Fernbach flasks (not shown) have inner angles between 50° and 70°. An inlay (not shown) for a Fernbach flask has an inner angle 18 of at least 50°.

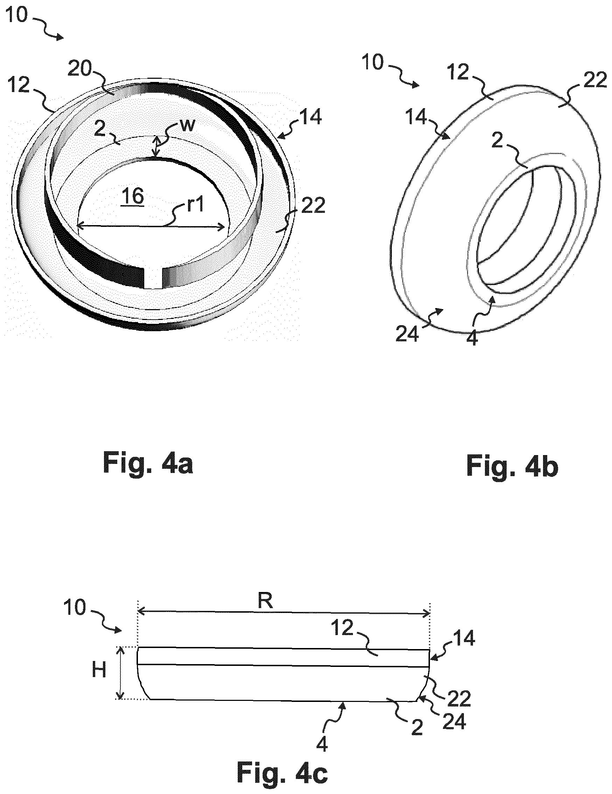

Fig. 4a, Fig. 4b, Fig. 4c illustrate another embodiment of the inlay 10. Fig. 4a, Fig. 4b give different perspective views of the inlay 10 according to the embodiment. Fig. 4c gives a side view.

The inlay 10 of Fig. 4a, Fig. 4b, Fig. 4c is formed with an opening 16.

The opening 16 makes it easier to fold the inlay 10 and it also improves the fit of the inlay 10 to the flask 30, in particular of the first surface 4 to the bottom inner surface 32 of the flask 30. Moreover, the opening 16 reduces the amount of material needed to fabricate the inlay 10.

The opening 16 may also be formed with any of the other embodiments.

The opening 16 comprises the center of the first surface 4. In the depicted embodiment, it is centered, but it may also be arranged slightly off-center. In the depicted embodiment, the opening 16 has a circular cross-section, but alternative cross-sections are possible, for as long as the amount of material of the bottom surface 2 is reduced sufficiently to improve the folding and the fit to the flask 30. A diameter r1 (Fig. 4a) of the opening 16 in the range from 1 - 3 cm has proven useful for this purpose. In case of a non-circular cross section, a width thereof is selected in the same range.

The intermediate portion 22 of the inlay 10 has an outward-facing surface 24, with a convex (i.e., outward-curved) shape, facing downward and outward. The curvature of the outward-facing surface 24 (Figs. 4b, 4c) is matched to the curvature of a typical lab flask 30 at the transition from its bottom inner wall 32 to its side inner walls 34. More specifically, the curvature is in the range from 6 mm to 6 cm, matched to the respective curvature of Erlenmeyer flasks 30 according to ISO 1773 or ISO 24450.

A corresponding outward-facing surface 24 may also be formed on any of the other embodiments.

The diameter R (cf. Fig. 4c) of the inlay 10 is in the range of 4 to 30 cm, matched to the maximum inner diameter of Erlenmeyer flasks 30 according to ISO 1773 or ISO 24450. Typically, the diameter R is selected with a value of 1 to 5 mm larger than an inner diameter of the flask 30 at its widest position.

The height H (cf. Fig. 4c) of the inlay 10 is in the range from 0.5 cm to 10 cm, depending on the size of the flask. Within this range, a smaller height H improves the folding, whereas a larger height H improves the adhesion to the side inner wall of the flask 30. The range from 0.5 cm to 6 cm ensures the balancing of both properties.

The width w of the ring-shaped bottom portion of the inlay 10 is approximately 1 cm in case of an inlay for a 250 ml Erlenmeyer flask.

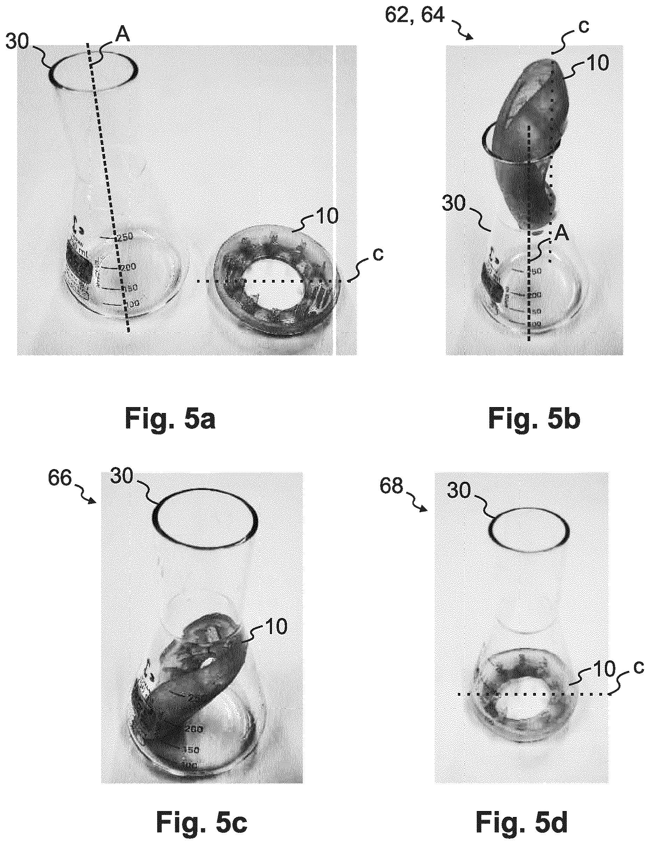

Fig. 5a, Fig. 5b, Fig. 5c, Fig. 5d illustrate a method for inserting an inlay 10 into a flask 30.

Fig. 5a shows the inlay 10 outside of the flask 30, as is the case at the beginning of the method. The flask 30 is an Erlenmeyer flask 30 with an axis A.

Fig. 5b shows the folding of the inlay 10 with respect to the crease line c.

Fig. 5b further shows that the inlay 10 with its crease line c is aligned with the axis A of the flask 30.

Fig. 5c shows the inlay 10 and the flask 30, after the inlay 10 has been inserted with its crease line c aligned with the axis A of the flask 30 (as depicted in Fig. 5b). The inlay 10 is arranged fully below the neck portion of the flask 30.

In Fig. 5c, the inlay 10 is being unfolded. In the depicted embodiment, the inlay 10 is an integral piece of elastomer, more specifically of silicone, formed by molding. As the forces applied to the inlay 10 during the insertion into the flask 30 (Fig. 5b) are released in Fig. 5c, the elastomer provides a restoring force driving the inlay 10 back into its original shape as depicted in Fig. 5a.

In alternative embodiments (not shown), the inlay 10 is not elastic, i.e., it does not comprise an elastomer. In corresponding embodiments, an additional action is required to unfold the inlay 10 inside of the flask 30. In other words, a machine, a tool or a worker grasps into the flask 30 and unfolds the inlay 10.

Fig. 5d shows the inlay 10 in its final state in the flask 30 after the unfolding.

In some embodiments (not shown) the inlay 10 is fixed in the flask 30, for example with glue. In yet other embodiments (not shown) a portion of the inlay 10 is removed, by etching or ashing (i.e., heating the flask 30 to a temperature above a decomposition temperature of the material of the inlay 10).

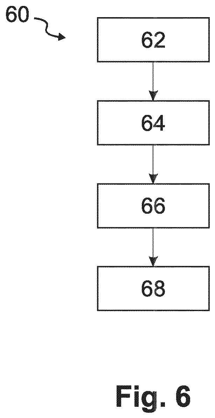

Fig. 6 illustrates a method 60 for inserting an inlay 10 into a flask 30 according to an alternative embodiment. The inlay 10 comprises a second flexible material and a baffle element 20.

The method 60 begins with folding 62 the second flexible material with respect to a crease line c.

The method 60 continues with aligning 64 the crease line c parallel to an axis A of the flask 30.

The method 60 continues with inserting 66 the folded inlay 10 into the flask 30 with the crease line c aligned with the axis A of the flask 30.

The method further comprises unfolding 68, unfolding the second flexible material, the inlay 10 in the flask 30, such that a downward-facing or downward-and-outward-facing first surface 4 and/or an outer surface 14 of the inlay 10 abuts an inner surface 32, 34 of the flask 30, and that the baffle element 20 extends into an inner volume 36 of the flask 30.

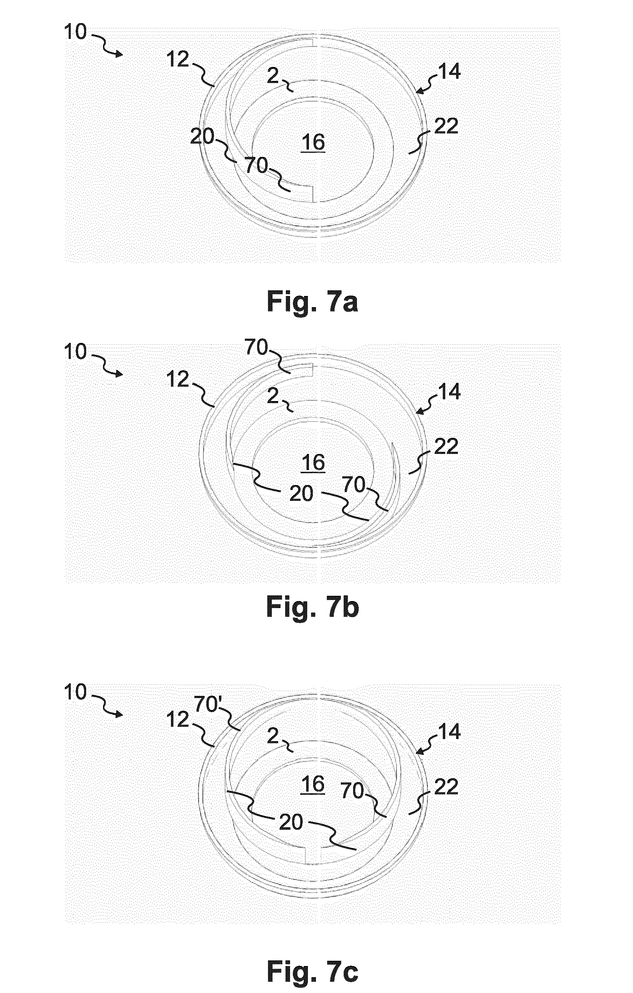

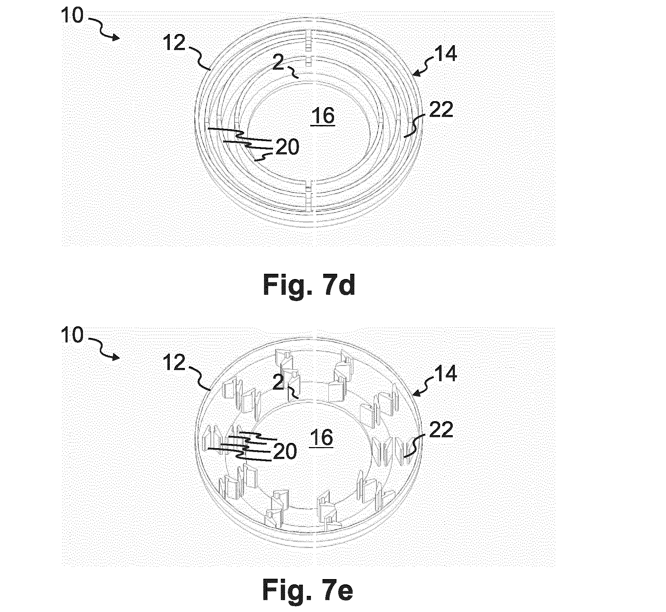

Fig. 7a, Fig. 7b, Fig. 7c, Fig. 7d, Fig. 7e give perspective views of inlays 10 according to various embodiments with a baffle element 20 extending upwards from the bottom portion 2.

In the embodiments of Fig. 7a, Fig. 7b, Fig. 7c, the baffle element 20 comprises a c-shaped, or half-moon-shaped element 70. The respective element has an extrusion shape, with a c-shaped, or half-moon-shaped horizontal basis in a horizontal plane. The extrusion refers to an extrusion along the vertical direction, resulting in the height of the respective baffle element 20.

The inlay 10 of Fig. 7a comprises a single c-shaped, or half-moon-shaped element 70.

The inlay 10 of Fig. 7b comprises two c-shaped, or half-moon-shaped elements 70, arranged opposite to each other, with an interruption along the azimuthal direction.

The inlay 10 of Fig. 7c comprises a single, extended c-shaped element 70' covering approximately 270° (i.e., more than 180°, and/or more than 230°) along the azimuthal direction. The c-shaped element 70' of Fig. 7c preferably comprises an interruption along the azimuthal direction.

In the embodiments of Fig. 7d, Fig. 7e, the baffle element 20 comprises fins extending upwards from the bottom portion 2. The fins have extrusion shapes with bases in a respective horizontal plane.

At least 4 or at least 8 or at least 10 fins are preferable. The embodiment of Fig. 7d comprises 12 fins. The embodiment of Fig. 7e comprises 40 fins.

Fig. 8a, Fig. 8b, Fig. 8c, Fig. 8d, Fig. 8e, Fig. 8f illustrate inlays 10 according to three embodiments with a baffle element 20 extending inwards from the side portion 12. Fig. 8b, Fig. 8c refer to the same embodiment. Fig. 8d, Fig. 8e, Fig. 8f refer to the same embodiment.

The inlay of Fig. 8a has a baffle element 20 with an inner contour, i.e., in a horizontal plane, having a rosette-shape. The depicted baffle element 20 has an inner contour according to an 8-leaved rosette. However, an inner contour according to a 3-, 4-, 5-, 6-, 7- or more leaved rosette is alternatively possible.

The inlay 10 of Fig. 8b, Fig. 8c and the inlay 10 of Fig. 8d, Fig. 8e, Fig. 8f has a baffle element 20 with a shape of a screw extending along an inner surface of the side portion 12.

The inlay 10 of Fig. 8b, Fig. 8c is adapted for a flask 30 with vertical inner side walls 34.

The inlay 10 of Fig. 8d, Fig. 8e, Fig. 8f is adapted for an Erlenmeyer flask 30.

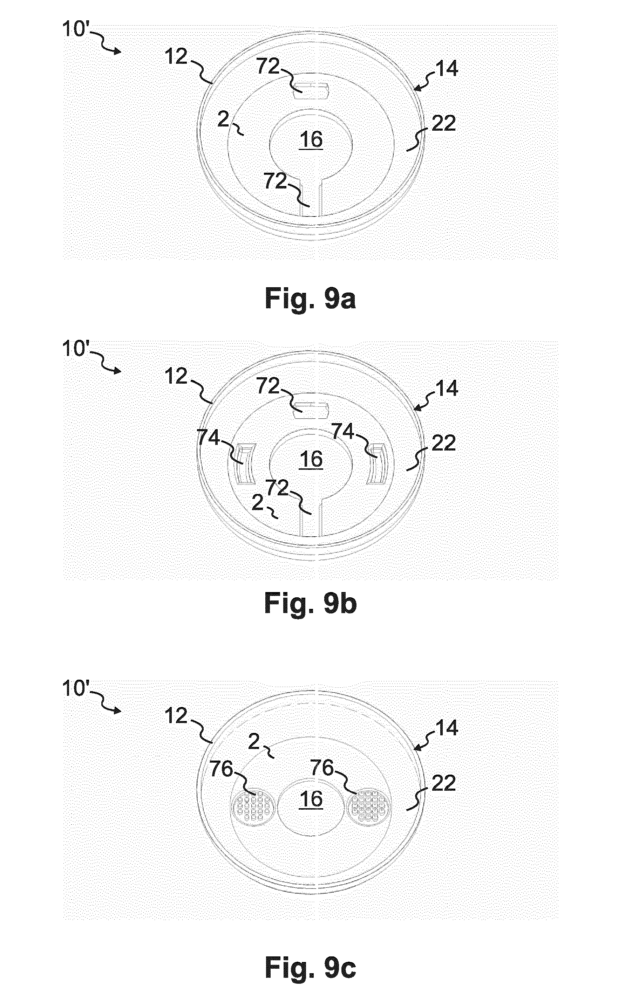

Fig. 9a, Fig. 9b, Fig. 9c illustrate additional elements, such as a second opening 72, a recess 74, or a light-emitting diode 76, respectively.

The bodies 10' depicted in Fig. 9a, Fig. 9b, Fig. 9c are similar to the inlays 10 described above, with a bottom portion 2, a side portion 12, and an intermediate portion 22. However, as compared to the respective inlays, the bodies 10' miss respective baffle elements. The bodies 10' may be combined with any of the baffle elements 20 described above, e.g., with a baffle element 20 from Fig. 7a, Fig. 7b, Fig. 7c, Fig. 7d, Fig. 7e, or from Fig. 8a, Fig. 8b, Fig. 8c, Fig. 8d, Fig. 8e, Fig. 8f to form an inlay 10. In other words, the additional elements of Fig. 9a, Fig. 9b, Fig. 9c (i.e., second opening 72, recess 74, or light-emitting diode 76) can be formed on the inlay 10 according to any of the embodiments described above.

The body 10' of Fig. 9a is formed with (second) openings 72, with horizontal cross sections and orientations along the vertical directions, i.e., perpendicular to the cross section of the respective opening 72.

When an inlay 10 with the body 10' of Fig. 9a is inserted into a flask 30, the openings 72 can be used to shine light through them into the inner volume 36 of the flask from below the flask 30, e.g., at an optode (e.g. for measuring pH and/or dissolved oxygen) arranged in the flask 30. Furthermore, the light can also directly shine into the solution when no optodes are applied in flask 30 to measure biomass concentration, particle concentration and/or particle size, fluorescent markers and/or particles or other optical signals. Therefore, the openings 72 permit in situ analysis of the solution in the flask 30.

The body 10' of Fig. 9b is formed with recesses 74 for a feeding pill or a feeding cartridge, providing nutrients to the culture in flask 30 without the need for a pump. Alternatively, the recess may be used to provide a well-defined position for a buffer pill or a buffer cartridge for regulating the pH value of the solution.

When an inlay 10 with the body 10' of Fig. 9b is inserted into a flask 30, the recesses 74 can be used to keep a feeding pill or a feeding cartridge at a controlled position with a well-defined local environment. Therefore, the recesses 74 permit a more reproducible cultivation of the microorganisms in the solution because feeding pills or discs do not aggregate/stick together due to the fluid movement (aggregation causes variable surface area and hence variable nutrient release via diffusion of nutrient into the fluid).

The body 10' of Fig. 9c is formed with light-emitting diodes 76 mounted thereon.

When an inlay 10 with the body 10' of Fig. 9c is inserted into a flask 30, the light-emitting diodes 76 permit cultivating phototrophic microorganisms, e.g. algae, providing a high light intensity directly to the fluid and hence optimized cultivation conditions in comparison to shake flasks that are illuminated traditionally from above or beneath the flask through the absorbing glass material of the flask.

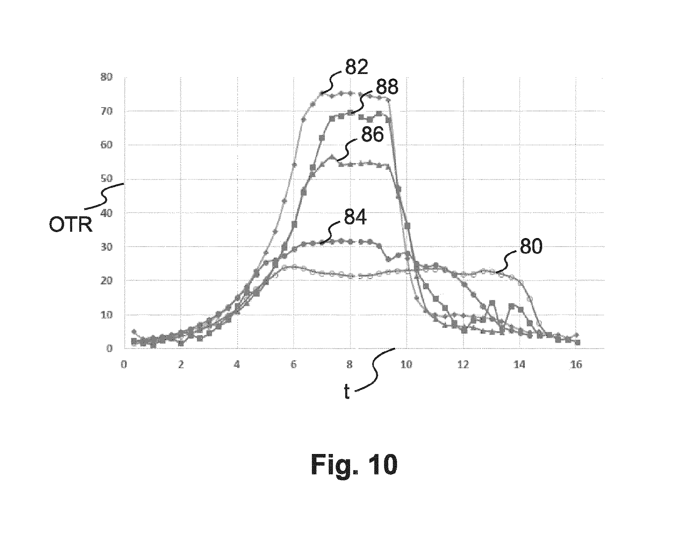

Fig. 10 compares the oxygen transfer rates OTR in mmol/(l*h) vs. time t in hours, for different flasks 30 with and without the inlay 10.

Oxygen transfer rate 80 is the one of a regular Erlenmeyer flask, such as a narrow-neck Erlenmeyer flask according to ISO 1773 or a wide-neck Erlenmeyer flask according to ISO 24450, i.e. without any baffle or baffle element. The oxygen transfer rate 80 is the lowest one measured with approx. 25 mmol/(l*h).

Oxygen transfer rate 82 is the one of a commercially available baffled flask. The oxygen transfer rate 82 is the highest one observed, thus providing a reference.

Oxygen transfer rate 84 is the one of an Erlenmeyer flask, wherein a body 10' without a baffle element 20 is inserted. The oxygen transfer rate 84 is similarly low as the oxygen transfer rate 80 of the Erlenmeyer flask.

Oxygen transfer rates 86, 88 are the ones of Erlenmeyer flasks, wherein a respective inlay 10 is inserted. More specifically, oxygen transfer rate 88 uses the inlay 10 of Fig. 7c, and oxygen transfer rate 86 uses the inlay 10 of Fig. 7e. The oxygen transfer rates 86, 88 are significantly improved as compared to the oxygen transfer rate 80 of the Erlenmeyer flask or to the oxygen transfer rate 84 of the body 10'.

To summarize Fig. 10, the inlay 10 with the baffle element 20 improves the oxygen transfer rate OTR to a degree similar to the one of a baffled flask.

### LIST OF REFERENCE SIGNS

- 10: inlay
- 30: flask
- 2: bottom portion of inlay
- 4: downward-facing or downward-and-outward-facing first surface
- 12: side portion of inlay
- 14: outer surface
- 22: intermediate portion of inlay
- 20: baffle element
- a: reference line
- 18: inner angle
- c: crease line
- 28: vertical plane
- t1: thickness of bottom portion
- t2: thickness of side portion
- 32: bottom inner surface of the flask
- 34: side inner surface of the flask
- 36: inner volume of the flask
- 16: opening
- 24: outward-facing surface of the intermediate portion of inlay
- r1: radius of opening
- w: width of the ring-shaped bottom portion
- R: radius of inlay
- H: height of inlay
- A: axis of flask
- 62: folding
- 64: aligning the crease line parallel to the axis of the flask
- 66: inserting the folded inlay into the flask
- 68: unfolding the inlay
- 70: c-shaped or half-moon-shaped element
- 72: second opening
- 74: recess
- 76: light emitting diode
- r: oxygen-transfer rate (mmol/(l*h))
- t: time (hours)

## Claims

1. An inlay (10) for a flask (30), the inlay (10) comprising:
a bottom portion (2) comprising a downward-facing or downward-and-outward-facing first surface (4) ;
a side portion (12) comprising an outer surface (14), wherein the outer surface (14) faces outwards with respect to a vertical reference line (a) intersecting the inlay (10);
an intermediate portion (22) mechanically connecting the bottom portion (2) and the side portion (12); and
a baffle element (20) extending away from the bottom portion (2), from the side portion (12), and/or from the intermediate portion (22);
wherein an inner angle (18) between the first surface (4) and the outer surface (14) is at least 50°, wherein the inner angle (18) refers to an angle on the side of the vertical reference line (a) in a vertical cross section through the inlay (10); and
wherein the bottom portion (2) in a vicinity of the intermediate portion (22), the intermediate portion (22), and/or the side portion (12) of the inlay (10) comprises a first flexible material to allow for bending said side portion (12) inwards;
wherein the inlay (10) is adapted to allow for folding the inlay (10) with respect to a horizontal crease line (c) to insert the inlay (10) into the flask (30); and
wherein the inlay (10) comprises, along a vertical plane (28) comprising the horizontal crease line (c), a second flexible material to allow for said folding.

2. The inlay (10) according to claim 1,
wherein a ratio of a height (H) of the inlay (10) along the vertical direction and a maximum width (R) of the inlay (10) in a horizontal direction is at most 0.5, or at most 0.4, or at most one third; and/or
wherein a height (H) of the inlay (10) is at most 6 cm, or at most 5 cm, or at most 4 cm; and/or
wherein a height (H) of the inlay (10) is at least 0.5 cm, or at least 0.75 cm, or at least 1 cm; and/or
material compositions of the first flexible material and the second flexible material are identical; and/or
wherein the inlay (10) is an integral part, such as an integral part formed by molding and/or an integral part composed of a flexible material providing the first flexible material and/or the second flexible material; and/or
wherein a surface of the inlay (10) is hydrophilic; and/or
wherein a surface of the bottom portion (2) and/or of the side portion (12) and/or of the intermediate portion (22) and/or of the baffle element (20) and/or of the first flexible material and/or of the second flexible material is hydrophilic.

3. The inlay (10) according to claim 1 or 2,
wherein the intermediate portion (22) is arranged between the bottom portion (2) and the side portion (12); and/or
wherein the intermediate portion (22) or an outward-facing surface (24) of the intermediate portion (22) extends from the outer surface (14) to the first surface (4), in particular, from a bottom edge of the outer surface (14) and/or to an outer edge of the first surface (4),
wherein, optionally, the outward-facing surface (24) of the intermediate portion (22) comprises an orientation with a downward component and/or with an outward component; and/or
wherein, optionally, the outward-facing surface (24) of the intermediate portion (22) comprises a convex shape, in particular, with a radius in a range from 6 mm to 6 cm.

4. The inlay (10) according to any of the preceding claims,
wherein the first flexible material is a first elastomer;
wherein the first elastomer is adapted to promote a force-fit connection of the first surface (4) to the bottom inner surface (32) of the flask (30) and/or to promote a force-fit connection of the outer surface (14) to the side inner surface (34) of the flask (30);
wherein, optionally, the first elastomer is adapted to, upon the bending the side portion (12) inwards, provide an elastic restoration force against the bending, said restoration force promoting said force-fit connection of the side portion (12) to the side inner surface (34) of the flask (30) and/or of the first surface (4) to the bottom inner surface (32) of the flask (30);
and/or:
wherein the second flexible material is a second elastomer;
wherein, optionally, the second elastomer is adapted to allow for said folding, and is adapted to unfold the inlay (10) upon said folding.

5. The inlay (10) according to any of the preceding claims,
wherein the downward-facing or downward-and-outward-facing first surface (4) is a downward-facing bottom surface (4) of the bottom portion (2); and/or
wherein, in a horizontal plane intersecting the bottom portion (2) and/or the first surface (4) and/or the intermediate portion (22), a shape of the bottom portion (2) and/or a shape of the first surface (4) and/or a shape of the intermediate portion (22) is a ring segment or a ring; and/or
wherein, in a horizontal plane intersecting the bottom portion (2) and/or the first surface (4), a shape of the bottom portion (2) and/or a shape of the first surface (4) is a ring segment or a ring, and wherein a width (w) of the ring segment or of the ring is at least 2 millimeters, or at least 4 mm, or at least 6 mm.

6. The inlay (10) according to any of the preceding claims,
wherein the bottom portion (2) and/or the first surface (4) comprises an opening (16), in particular, comprising its center/their centers or centered in its center/their centers,
wherein optionally, the opening (16) has a width or a diameter (r1) of at least 1 cm or of at least 2 cm or of at least 3 cm, and/or
wherein, optionally, the opening (16) has a circular shape and/or the opening is centered in the first surface (4).

7. The inlay (10) according to claim 4, optionally in combination with any other of the preceding claims,
wherein the inlay (10) is an integral part composed of an elastomer material providing the first elastomer and the second elastomer; and/or
wherein the first elastomer and/or the second elastomer comprises or is rubber or silicone; and/or
wherein material compositions of the first elastomer and the second elastomer are identical; and/or
wherein the intermediate portion (22) and/or the side portion (12) comprise(s) or is/are composed of the second elastomer; and/or
wherein the bottom portion (2) comprises or is composed of the first elastomer,
wherein, optionally the side portion (12) and/or the intermediate portion (22) comprises or is composed of the first elastomer, and/or
wherein, optionally, the baffle element (20) comprises or is composed of the first elastomer.

8. The inlay (10) according to any of the preceding claims,
wherein a thickness (t1) of the bottom portion (2), in particular along the vertical direction, is at most 6 mm, or at most 5 mm, or at most 4 mm; and/or
wherein a thickness (t2) of the side portion (12), in particular along a radial direction with respect to the vertical reference line (a), is at most 6 mm, or at most 5 mm, or at most 4 mm; and/or
wherein a thickness of the intermediate portion (22) is at most 6 mm, or at most 5 mm, or at most 4 mm; and/or
wherein the first surface (4) is flat and/or extends in a horizontal plane.

9. The inlay (10) according to any of the preceding claims,
wherein the inner angle (18) is at least 60°, or at least 70°, or at least 75°, or at least 80°, or at least 85°, or at least 90°, or more than 90°, or at least 91°, or more than 95°; and/or
wherein a height of the outer surface (14) along the vertical direction is at least 2 mm, or at least 4 mm, or at least 6 mm; and/or
wherein a width or a diameter (R) of the inlay (10) in the horizontal direction is in a range from 4 cm to 30 cm; and/or
wherein the vertical reference line (a) extends through a center of the inlay (10) and/or of the bottom portion (2) and/or of the side portion (12) and/or of the intermediate portion (22); and/or
wherein the horizontal crease line (c) extends through the bottom portion (2) and/or through the intermediate portion (22) and/or through the first surface (4), in particular, through a center thereof; and/or
wherein the vertical cross section and/or the vertical plane (28) comprise(s) the vertical reference line (a); and/or
wherein the outer surface (14) or at least a section of the outer surface (14) extends along a side of a reference cylinder, wherein an axis of the reference cylinder is aligned with the vertical reference line (a), and wherein, optionally, the first surface (4) and/or the bottom portion (2) and/or the intermediate portion (22) and/or the baffle element (20) is/are fully comprised in the reference cylinder; and/or
wherein the bending the side portion (12) inwards bends the side portion (12) towards the vertical reference line (a) and/or reduces the inner angle (18), wherein, optionally, the elastic restoration force acts to bend the side portion (12) away from the vertical reference line (a) and/or to increase the inner angle (18).

10. The inlay (10) according to any of the preceding claims,
wherein the baffle element (20) comprises or is composed of an elastomer; and/or
wherein the baffle element (20) extends upwards from the bottom portion (2); and/or
wherein the baffle element (20) extends inwards from the side portion (12); and/or
wherein the baffle element (20) is recessed above the horizontal crease line (c).

11. The inlay (10) according to any of the preceding claims, which further comprises:
a second opening (72) adapted for receiving an optode or for permitting a line of sight from below the flask (30) to the inner volume (36) of the flask (30), in particular, wherein the second opening (72) has an extrusion shape corresponding to an extrusion of a base in a horizontal plane along the vertical direction; and/or
a recess (74) adapted to receive a feeding pill or a feeding cartridge or a buffer pill or a buffer cartridge, in particular, wherein the recess faces upwards or inwards, such as from the bottom portion (2) and/or from the intermediate portion (22); and/or
a light-emitting diode (76) mounted to the inlay (10), in particular, to the bottom portion (2) and/or to the intermediate portion (22).

12. The inlay (10) according to any of the preceding claims, wherein the flask (30) is an Erlenmeyer flask (30), in particular a wide-neck or a narrow-neck Erlenmeyer flask (30), such as a narrow-neck Erlenmeyer flask (30) according to ISO 1773 or a wide-neck Erlenmeyer flask (30) according to ISO 24450.

13. A method (60) for inserting an inlay (10) into a flask (30), the inlay (10) comprising a flexible material and a baffle element (20), the method comprising:
folding (62) the flexible material with respect to a crease line (c);
aligning (64) the crease line (c) parallel to an axis (A) of the flask (30);
inserting (66) the folded inlay (10) into the flask (30) with the crease line (c) aligned with the axis (A) of the flask (30); and
unfolding (68), unfolding the flexible material, the inlay (10) in the flask (30), such that a downward-facing or downward-and-outward-facing first surface (4) and/or an outer surface (14) of the inlay (10) abuts an inner surface (32, 34) of the flask (30), and that the baffle element (20) extends into an inner volume (36) of the flask (30).

14. The method of claim 13,
wherein the flexible material is an elastomer, wherein, optionally, the elastomer promotes the unfolding (68) the inlay (10) in the flask (30);
wherein, optionally, the inlay (10) is unfolded by the elastomer such that the crease line (c) is orientated parallel to a bottom surface (34) of the flask (30) and/or orthogonal to the axis (A) of the flask (30); and/or
wherein the inlay (10) is unfolded such that the inlay (10) is fully positioned below a neck portion of the flask (30); and/or
wherein the method further comprises fixing the inlay (10) in the flask (30) and/or removing a section of the inlay (10) in the flask (30), such as by etching or ashing.

15. The method of claim 13 or 14, wherein the inlay (10) comprises a bottom portion (2) comprising the first surface (4), a side portion (12) comprising the outer surface (14), an intermediate portion (22) mechanically connecting the bottom portion (2) and the side portion (12), and an additional flexible material, such as an additional elastomer, wherein the unfolding comprises:
arranging the first surface (4) to abut a bottom inner surface (32) of the flask (30); and
arranging the outer surface (14) to abut a side inner surface (34) of the flask (30);
and optionally, in embodiments with the first elastomer: providing, by the first elastomer, a force-fit connection of the first surface (4) to the bottom inner surface (32) of the flask (30) and/or a force-fit connection of the outer surface (14) to the side inner surface (34) of the flask (30).

## Patentansprüche

1. Einsatz (10) für ein Gefäß (30), wobei der Einsatz (10) Folgendes aufweist:
einen unteren Abschnitt (2), der eine nach unten gerichtete oder nach unten und nach außen gerichtete erste Oberfläche (4) aufweist;
einen Seitenabschnitt (12), der eine äußere Oberfläche (14) aufweist, wobei die äußere Oberfläche (14) bezogen auf eine vertikale Bezugslinie (a), die den Einsatz (10) schneidet, nach außen gerichtet ist;
einen Zwischenabschnitt (22), der den unteren Abschnitt (2) und den Seitenabschnitt (12) mechanisch verbindet; und
ein Ablenkelement (20), das sich von dem unteren Abschnitt (2), von dem Seitenabschnitt (12) und/oder von dem Zwischenabschnitt (22) weg erstreckt;
wobei ein Innenwinkel (18) zwischen der ersten Oberfläche (4) und der äußeren Oberfläche (14) mindestens 50° beträgt, wobei sich der Innenwinkel (18) auf einen Winkel auf der Seite der vertikalen Bezugslinie (a) in einem vertikalen Querschnitt durch den Einsatz (10) bezieht; und
wobei der untere Abschnitt (2) in der Nähe des Zwischenabschnitts (22), der Zwischenabschnitts (22) und/oder der Seitenabschnitt (12) des Einsatzes (10) ein erstes flexibles Material aufweist, um ein Biegen des Seitenabschnitts (12) nach innen zu ermöglichen;
wobei der Einsatz (10) angepasst ist, um ein Falten des Einsatzes (10) bezogen auf eine horizontale Faltlinie (c) zu ermöglichen, um den Einsatz (10) in das Gefäß (30) einzubringen; und
wobei der Einsatz (10) entlang einer vertikalen Ebene (28), die die horizontale Faltlinie (c) aufweist, ein zweites flexibles Material aufweist, um das Falten zu ermöglichen.

2. Einsatz (10) nach Anspruch 1,
wobei ein Verhältnis einer Höhe (H) des Einsatzes (10) entlang der vertikalen Richtung und einer maximalen Breite (R) des Einsatzes (10) in einer horizontalen Richtung höchstens 0,5 oder höchstens 0,4 oder höchstens ein Drittel beträgt; und/oder
wobei eine Höhe (H) des Einsatzes (10) höchstens 6 cm oder höchstens 5 cm oder höchstens 4 cm beträgt; und/oder
wobei eine Höhe (H) des Einsatzes (10) mindestens 0,5 cm oder mindestens 0,75 cm oder mindestens 1 cm beträgt; und/oder
Materialzusammensetzungen des ersten flexiblen Materials und des zweiten flexiblen Materials identisch sind; und/oder
wobei der Einsatz (10) ein monolithisches Teil ist, wie etwa ein monolithisches Teil, das durch Formen gebildet wird, und/oder ein monolithisches Teil, das aus einem flexiblen Material besteht, das das erste flexible Material und/oder das zweite flexible Material bereitstellt; und/oder
wobei eine Oberfläche des Einsatzes (10) hydrophil ist; und/oder
wobei eine Oberfläche des unteren Abschnitts (2) und/oder des Seitenabschnitts (12) und/oder des Zwischenabschnitts (22) und/oder des Ablenkelements (20) und/oder des ersten flexiblen Materials und/oder des zweiten flexiblen Materials hydrophil ist.

3. Einsatz (10) nach Anspruch 1 oder 2,
wobei der Zwischenabschnitt (22) zwischen dem unteren Abschnitt (2) und dem Seitenabschnitt (12) angeordnet ist; und/oder
wobei sich der Zwischenabschnitt (22) oder eine nach außen gerichtete Oberfläche (24) des Zwischenabschnitts (22) von der äußeren Oberfläche (14) zu der ersten Oberfläche (4) erstreckt, insbesondere von einer unteren Kante der äußeren Oberfläche (14) und/oder zu einer äußeren Kante der ersten Oberfläche (4),
wobei optional die nach außen gerichtete Oberfläche (24) des Zwischenabschnitts (22) eine Ausrichtung mit einer nach unten gerichteten Komponente und/oder mit einer nach außen gerichteten Komponente aufweist; und/oder
wobei optional die nach außen gerichtete Oberfläche (24) des Zwischenabschnitts (22) eine konvexe Form aufweist, insbesondere mit einem Radius in einem Bereich von 6 mm bis 6 cm.

4. Einsatz (10) nach einem der vorhergehenden Ansprüche,
wobei das erste flexible Material ein erstes Elastomer ist;
wobei das erste Elastomer angepasst ist, um eine kraftschlüssige Verbindung der ersten Oberfläche (4) mit der unteren inneren Oberfläche (32) des Gefäßes (30) zu unterstützen und/oder um eine kraftschlüssige Verbindung der äußeren Oberfläche (14) mit der seitlichen inneren Oberfläche (34) des Gefäßes (30) zu unterstützen;
wobei optional das erste Elastomer eingerichtet ist, um beim Biegen des Seitenabschnitts (12) nach innen eine elastische Rückstellkraft gegen das Biegen bereitzustellen, wobei die Rückstellkraft die kraftschlüssige Verbindung des Seitenabschnitts (12) mit der seitlichen inneren Oberfläche (34) des Gefäßes (30) und/oder der ersten Oberfläche (4) mit der unteren inneren Oberfläche (32) des Gefäßes (30) unterstützt;
und/oder:
wobei das zweite flexible Material ein zweites Elastomer ist;
wobei optional das zweite Elastomer eingerichtet ist, um das Falten zu ermöglichen, und eingerichtet ist, um den Einsatz (10) beim Falten zu entfalten.

5. Einsatz (10) nach einem der vorhergehenden Ansprüche,
wobei die nach unten gerichtete oder nach unten und nach außen gerichtete erste Oberfläche (4) eine nach unten gerichtete untere Oberfläche (4) des unteren Abschnitts (2) ist; und/oder
wobei in einer horizontalen Ebene, die den unteren Abschnitt (2) und/oder die erste Oberfläche (4) und/oder den Zwischenabschnitt (22) schneidet, eine Form des unteren Abschnitts (2) und/oder eine Form der ersten Oberfläche (4) und/oder eine Form des Zwischenabschnitts (22) ein Ringsegment oder ein Ring ist; und/oder
wobei in einer horizontalen Ebene, die den unteren Abschnitt (2) und/oder die erste Oberfläche (4) schneidet, eine Form des unteren Abschnitts (2) und/oder eine Form der ersten Oberfläche (4) ein Ringsegment oder ein Ring ist, und wobei eine Breite (w) des Ringsegments oder des Rings mindestens 2 Millimeter oder mindestens 4 mm oder mindestens 6 mm beträgt.

6. Einsatz (10) nach einem der vorhergehenden Ansprüche,
wobei der untere Abschnitt (2) und/oder die erste Oberfläche (4) eine Öffnung (16) aufweist, insbesondere dessen/ihre Mittelpunkt(e), oder zentriert an seinem Mittelpunkt/ihrem Mittelpunkt/ihren Mittelpunkten,
wobei optional die Öffnung (16) eine Breite oder einen Durchmesser (r1) von mindestens 1 cm oder von mindestens 2 cm oder von mindestens 3 cm aufweist, und/oder
wobei optional die Öffnung (16) eine kreisförmige Form aufweist und/oder die Öffnung in der ersten Oberfläche (4) zentriert ist.

7. Einsatz (10) nach Anspruch 4, optional in Kombination mit einem anderen der vorhergehenden Ansprüche,
wobei der Einsatz (10) ein monolithischer Teil ist, der aus einem Elastomermaterial besteht, das das erste Elastomer und das zweite Elastomer bereitstellt; und/oder
wobei das erste Elastomer und/oder das zweite Elastomer Gummi oder Silikon aufweist oder ist; und/oder
wobei Materialzusammensetzungen des ersten Elastomers und des zweiten Elastomers identisch sind; und/oder
wobei der Zwischenabschnitt (22) und/oder der Seitenabschnitt (12) das zweite Elastomer aufweist/aufweisen oder daraus besteht/bestehen; und/oder
wobei der untere Abschnitt (2) das erste Elastomer aufweist oder daraus besteht,
wobei optional der Seitenabschnitt (12) und/oder der Zwischenabschnitt (22) das erste Elastomer aufweist oder daraus besteht, und/oder
wobei optional das Ablenkelement (20) das erste Elastomer aufweist oder daraus besteht.

8. Einsatz (10) nach einem der vorhergehenden Ansprüche,
wobei eine Dicke (t1) des unteren Abschnitts (2), insbesondere entlang der vertikalen Richtung, höchstens 6 mm oder höchstens 5 mm oder höchstens 4 mm beträgt; und/oder
wobei eine Dicke (t2) des Seitenabschnitts (12), insbesondere entlang einer radialen Richtung bezogen auf die vertikale Bezugslinie (a), höchstens 6 mm oder höchstens 5 mm oder höchstens 4 mm beträgt; und/oder
wobei eine Dicke des Zwischenabschnitts (22) höchstens 6 mm oder höchstens 5 mm oder höchstens 4 mm beträgt; und/oder
wobei die erste Oberfläche (4) flach ist und/oder sich in einer horizontalen Ebene erstreckt.

9. Einsatz (10) nach einem der vorhergehenden Ansprüche,
wobei der Innenwinkel (18) mindestens 60° oder mindestens 70° oder mindestens 75° oder mindestens 80° oder mindestens 85° oder mindestens 90° oder mehr als 90° oder mindestens 91° oder mehr als 95° beträgt; und/oder
wobei eine Höhe der äußeren Oberfläche (14) entlang der vertikalen Richtung mindestens 2 mm oder mindestens 4 mm oder mindestens 6 mm beträgt; und/oder
wobei eine Breite oder ein Durchmesser (R) des Einsatzes (10) in der horizontalen Richtung in einem Bereich von 4 cm bis 30 cm liegt; und/oder
wobei sich die vertikale Bezugslinie (a) durch einen Mittelpunkt des Einsatzes (10) und/oder des unteren Abschnitts (2) und/oder des Seitenabschnitts (12) und/oder des Zwischenabschnitts (22) erstreckt; und/oder
wobei sich die horizontale Faltlinie (c) durch den unteren Abschnitt (2) und/oder durch den Zwischenabschnitt (22) und/oder durch die erste Oberfläche (4) erstreckt, insbesondere durch einen Mittelpunkt davon; und/oder
wobei der vertikale Querschnitt und/oder die vertikale Ebene (28) die vertikale Bezugslinie (a) aufweist; und/oder
wobei sich die äußere Oberfläche (14) oder zumindest ein Abschnitt der äußeren Oberfläche (14) entlang einer Seite eines Bezugszylinders erstreckt, wobei eine Achse des Bezugszylinders mit der vertikalen Bezugslinie (a) ausgerichtet ist, und wobei optional die erste Oberfläche (4) und/oder der untere Abschnitt (2) und/oder der Zwischenabschnitt (22) und/oder das Ablenkelement (20) vollständig in dem Bezugszylinder umfasst ist/sind; und/oder
wobei das Biegen des Seitenabschnitts (12) nach innen den Seitenabschnitt (12) in Richtung der vertikalen Bezugslinie (a) biegt und/oder den Innenwinkel (18) verringert,
wobei optional die elastische Rückstellkraft wirkt, um den Seitenabschnitt (12) von der vertikalen Bezugslinie (a) weg zu biegen und/oder den Innenwinkel (18) zu vergrößern.

10. Einsatz (10) nach einem der vorhergehenden Ansprüche,
wobei das Ablenkelement (20) ein Elastomer aufweist oder daraus besteht; und/oder
wobei sich das Ablenkelement (20) von dem unteren Abschnitt (2) nach oben erstreckt; und/oder
wobei sich das Ablenkelement (20) von dem Seitenabschnitt (12) nach innen erstreckt; und/oder
wobei das Ablenkelement (20) über der horizontalen Faltlinie (c) ausgenommen ist.

11. Einsatz (10) nach einem der vorhergehenden Ansprüche, welcher ferner aufweist:
eine zweite Öffnung (72), die eingerichtet ist, um eine Optode aufzunehmen oder um eine Sichtlinie von unterhalb des Gefäßes (30) zu dem Innenvolumen (36) des Gefäßes (30) zu ermöglichen, insbesondere wobei die zweite Öffnung (72) eine Extrusionsform aufweist, die einer Extrusion einer Basis in einer horizontalen Ebene entlang der vertikalen Richtung entspricht; und/oder
eine Aussparung (74), die eingerichtet ist, um ein Nährstoffpellet oder eine Nährstoffkartusche oder eine Puffertablette oder eine Pufferkartusche aufzunehmen, insbesondere wobei die Aussparung nach oben oder nach innen gerichtet ist, wie etwa von dem unteren Abschnitt (2) und/oder von dem Zwischenabschnitt (22); und/oder eine Leuchtdiode (76), die an dem Einsatz (10) montiert ist, insbesondere an dem unteren Abschnitt (2) und/oder an dem Zwischenabschnitt (22).

12. Einsatz (10) nach einem der vorhergehenden Ansprüche, wobei das Gefäß (30) ein Erlenmeyerkolben (30) ist, insbesondere ein Weithals- oder ein Enghals-Erlenmeyerkolben (30), wie etwa ein Enghals-Erlenmeyerkolben (30) gemäß ISO 1773 oder ein Weithals-Erlenmeyerkolben (30) gemäß ISO 24450.

13. Verfahren (60) zum Einbringen eines Einsatzes (10) in ein Gefäß (30), wobei der Einsatz (10) ein flexibles Material und ein Ablenkelement (20) aufweist, wobei das Verfahren Folgendes umfasst:
Falten (62) des flexiblen Materials bezogen auf eine Faltlinie (c);
Ausrichten (64) der Faltlinie (c) parallel zu einer Achse (A) des Gefäßes (30);
Einbringen (66) des gefalteten Einsatzes (10) in das Gefäß (30), wobei die Faltlinie (c) mit der Achse (A) des Gefäßes (30) ausgerichtet ist; und
Entfalten (68), wobei das flexible Material entfaltet wird, des Einsatzes (10) in dem Gefäß (30), so dass eine nach unten gerichtete oder nach unten und nach außen gerichtete erste Oberfläche (4) und/oder eine äußere Oberfläche (14) des Einsatzes (10) an einer inneren Oberfläche (32, 34) des Gefäßes (30) anliegt, und so dass sich das Ablenkelement (20) in ein Innenvolumen (36) des Gefäßes (30) erstreckt.

14. Verfahren nach Anspruch 13,
wobei das flexible Material ein Elastomer ist, wobei optional das Elastomer das Entfalten (68) des Einsatzes (10) in dem Gefäß (30) unterstützt;
wobei optional der Einsatz (10) durch das Elastomer entfaltet wird, so dass die Faltlinie (c) parallel zu einer unteren Oberfläche (34) des Gefäßes (30) und/oder orthogonal zu der Achse (A) des Gefäßes (30) ausgerichtet ist; und/oder
wobei der Einsatz (10) so entfaltet wird, dass der Einsatz (10) vollständig unter einem Halsabschnitt des Gefäßes (30) positioniert ist; und/oder
wobei das Verfahren ferner das Fixieren des Einsatzes (10) in dem Gefäß (30) und/oder das Entfernen eines Abschnitts des Einsatzes (10) in dem Gefäß (30), wie etwa durch Ätzen oder Veraschen, umfasst.

15. Verfahren nach Anspruch 13 oder 14, wobei der Einsatz (10) einen unteren Abschnitt (2) aufweist, aufweisend die erste Oberfläche (4), einen Seitenabschnitt (12), der die äußere Oberfläche (14) aufweist, einen Zwischenabschnitt (22), der den unteren Abschnitt (2) und den Seitenabschnitt (12) mechanisch verbindet, und ein zusätzliches flexibles Material, wie etwa ein zusätzliches Elastomer, wobei das Entfalten Folgendes umfasst:
Anordnen der ersten Oberfläche (4), um an einer unteren inneren Oberfläche (32) des Gefäßes (30) anzuliegen; und
Anordnen der äußeren Oberfläche (14), um an einer seitlichen inneren Oberfläche (34) des Gefäßes (30) anzuliegen;
und optional, in Ausführungsformen mit dem ersten Elastomer: Bereitstellen, durch das erste Elastomer, einer kraftschlüssigen Verbindung der ersten Oberfläche (4) mit der unteren inneren Oberfläche (32) des Gefäßes (30) und/oder einer kraftschlüssigen Verbindung der äußeren Oberfläche (14) mit der seitlichen inneren Oberfläche (34) des Gefäßes (30).

## Revendications

1. Insert (10) pour flacon (30), l'insert (10) comprenant :
une partie inférieure (2) comprenant une première surface (4) orientée vers le bas ou vers le bas et vers l'extérieur ;
une partie latérale (12) comprenant une surface extérieure (14), dans laquelle la surface extérieure (14) est orientée vers l'extérieur par rapport à une ligne de référence verticale (a) coupant l'insert (10) ;
une partie intermédiaire (22) reliant mécaniquement la partie inférieure (2) et la partie latérale (12) ; et
un élément déflecteur (20) s'étendant à partir de la partie inférieure (2), de la partie latérale (12) et/ou de la partie intermédiaire (22) ;
dans lequel un angle intérieur (18) entre la première surface (4) et la surface extérieure (14) est d'au moins 50°, dans lequel l'angle intérieur (18) fait référence à un angle du côté de la ligne de référence verticale (a) dans une section transversale verticale passant par l'insert (10) ; et
dans lequel la partie inférieure (2) à proximité de la partie intermédiaire (22), la partie intermédiaire (22), et/ou la partie latérale (12) de l'insert (10) comprend/comprennent un premier matériau flexible pour permettre la flexion de ladite partie latérale (12) vers l'intérieur ;
dans lequel l'insert (10) est adapté pour permettre le pliage de l'insert (10) par rapport à une ligne de pli horizontale (c) afin d'insérer l'insert (10) dans le flacon (30) ; et
dans lequel l'insert (10) comprend, le long d'un plan vertical (28) comprenant la ligne de pli horizontale (c), un deuxième matériau flexible pour permettre ledit pliage.

2. Insert (10) selon la revendication 1,
dans lequel un rapport entre la hauteur (H) de l'insert (10) dans la direction verticale et la largeur maximale (R) de l'insert (10) dans la direction horizontale est au plus égal à 0,5, ou au plus égal à 0,4, ou au plus égal à un tiers ; et/ou
dans lequel une hauteur (H) de l'insert (10) est au plus de 6 cm, ou au plus de 5 cm, ou au plus de 4 cm ; et/ou
dans lequel une hauteur (H) de l'insert (10) est d'au moins 0,5 cm, ou d'au moins 0,75 cm, ou d'au moins 1 cm ; et/ou
les compositions de matériaux du premier matériau flexible et du deuxième matériau flexible sont identiques ; et/ou
dans lequel l'insert (10) est une partie intégrante, telle qu'une partie intégrante formée par moulage et/ou une partie intégrante composée d'un matériau flexible fournissant le premier matériau flexible et/ou le deuxième matériau flexible ; et/ou
dans lequel une surface de l'insert (10) est hydrophile ; et/ou
dans lequel une surface de la partie inférieure (2) et/ou de la partie latérale (12) et/ou de la partie intermédiaire (22) et/ou de l'élément déflecteur (20) et/ou du premier matériau flexible et/ou du deuxième matériau flexible est hydrophile.

3. Insert (10) selon la revendication 1 ou 2,
dans lequel la partie intermédiaire (22) est agencée entre la partie inférieure (2) et la partie latérale (12) ; et/ou
dans lequel la partie intermédiaire (22) ou une surface orientée vers l'extérieur (24) de la partie intermédiaire (22) s'étend depuis la surface extérieure (14) jusqu'à la première surface (4), en particulier depuis un bord inférieur de la surface extérieure (14) et/ou jusqu'à un bord extérieur de la première surface (4),
dans lequel, éventuellement, la surface orientée vers l'extérieur (24) de la partie intermédiaire (22) comprend une orientation avec une composante vers le bas et/ou avec une composante vers l'extérieur ; et/ou
dans lequel, éventuellement, la surface orientée vers l'extérieur (24) de la partie intermédiaire (22) comprend une forme convexe, en particulier avec un rayon compris dans une plage allant de 6 mm à 6 cm.

4. Insert (10) selon l'une quelconque des revendications précédentes,
dans lequel le premier matériau flexible est un premier élastomère ;
dans lequel le premier élastomère est adapté pour favoriser une liaison par ajustement serré de la première surface (4) à la surface intérieure inférieure (32) du flacon (30) et/ou pour favoriser une liaison par ajustement serré de la surface extérieure (14) à la surface intérieure latérale (34) du flacon (30) ;
dans lequel, éventuellement, le premier élastomère est adapté pour, lors de la flexion de la partie latérale (12) vers l'intérieur, fournir une force de restauration élastique contre la flexion, ladite force de restauration favorisant ladite liaison par ajustement serré de la partie latérale (12) à la surface intérieure latérale (34) du flacon (30) et/ou de la première surface (4) à la surface intérieure inférieure (32) du flacon (30) ;
et/ou :
dans lequel le deuxième matériau flexible est un deuxième élastomère ;
dans lequel, éventuellement, le deuxième élastomère est adapté pour permettre ledit pliage et est adapté pour déplier l'insert (10) lors dudit pliage.

5. Insert (10) selon l'une quelconque des revendications précédentes,
dans lequel la première surface (4) orientée vers le bas ou vers le bas et vers l'extérieur est une surface inférieure (4) orientée vers le bas de la partie inférieure (2) ; et/ou
dans lequel, dans un plan horizontal coupant la partie inférieure (2) et/ou la première surface (4) et/ou la partie intermédiaire (22), une forme de la partie inférieure (2) et/ou une forme de la première surface (4) et/ou une forme de la partie intermédiaire (22) est un segment d'anneau ou un anneau ; et/ou
dans lequel, dans un plan horizontal coupant la partie inférieure (2) et/ou la première surface (4), une forme de la partie inférieure (2) et/ou une forme de la première surface (4) est un segment d'anneau ou un anneau, et dans lequel une largeur (w) du segment d'anneau ou de l'anneau est d'au moins 2 millimètres, ou d'au moins 4 mm, ou d'au moins 6 mm.

6. Insert (10) selon l'une quelconque des revendications précédentes,
dans lequel la partie inférieure (2) et/ou la première surface (4) comprend une ouverture (16), en particulier comprenant son centre/leurs centres ou centrée dans son centre/leurs centres,
dans lequel, éventuellement, l'ouverture (16) a une largeur ou un diamètre (r1) d'au moins 1 cm ou d'au moins 2 cm ou d'au moins 3 cm, et/ou
dans lequel, éventuellement, l'ouverture (16) a une forme circulaire et/ou l'ouverture est centrée dans la première surface (4).

7. Insert (10) selon la revendication 4, éventuellement en combinaison avec l'une quelconque des revendications précédentes,
dans lequel l'insert (10) est une partie intégrante composée d'un matériau élastomère fournissant le premier élastomère et le deuxième élastomère ; et/ou
dans lequel le premier élastomère et/ou le deuxième élastomère comprend/comprennent ou est/sont du caoutchouc ou du silicone ; et/ou dans lequel les compositions de matériaux du premier élastomère et du deuxième élastomère sont identiques ; et/ou
dans lequel la partie intermédiaire (22) et/ou la partie latérale (12) comprend/comprennent ou est/sont composée(s) du deuxième élastomère ; et/ou
dans lequel la partie inférieure (2) comprend ou est composée du premier élastomère, dans lequel, éventuellement, la partie latérale (12) et/ou la partie intermédiaire (22) comprend ou est composée du premier élastomère, et/ou
dans lequel, éventuellement, l'élément déflecteur (20) comprend ou est composé du premier élastomère.

8. Insert (10) selon l'une quelconque des revendications précédentes,
dans lequel une épaisseur (t1) de la partie inférieure (2), en particulier dans la direction verticale, est au plus de 6 mm, ou au plus de 5 mm, ou au plus de 4 mm ; et/ou
dans lequel une épaisseur (t2) de la partie latérale (12), en particulier dans une direction radiale par rapport à la ligne de référence verticale (a), est au plus de 6 mm, ou au plus de 5 mm, ou au plus de 4 mm ; et/ou
dans lequel une épaisseur de la partie intermédiaire (22) est au plus de 6 mm, ou au plus de 5 mm, ou au plus de 4 mm ; et/ou
dans lequel la première surface (4) est plane et/ou s'étend dans un plan horizontal.

9. Insert (10) selon l'une quelconque des revendications précédentes,
dans lequel l'angle intérieur (18) est d'au moins 60°, ou d'au moins 70°, ou d'au moins 75°, ou d'au moins 80°, ou d'au moins 85°, ou d'au moins 90°, ou supérieur à 90°, ou d'au moins 91°, ou supérieur à 95° ; et/ou
dans lequel une hauteur de la surface extérieure (14) dans la direction verticale est d'au moins 2 mm, ou d'au moins 4 mm, ou d'au moins 6 mm ; et/ou
dans lequel une largeur ou un diamètre (R) de l'insert (10) dans la direction horizontale est comprise dans une plage allant de 4 cm à 30 cm ; et/ou
dans lequel la ligne de référence verticale (a) s'étend à travers un centre de l'insert (10) et/ou de la partie inférieure (2) et/ou de la partie latérale (12) et/ou de la partie intermédiaire (22) ; et/ou
dans lequel la ligne de pli horizontale (c) s'étend à travers la partie inférieure (2) et/ou à travers la partie intermédiaire (22) et/ou à travers la première surface (4), en particulier à travers un centre de celle(s)-ci ; et/ou
dans lequel la section transversale verticale et/ou le plan vertical (28) comprend/comprennent la ligne de référence verticale (a) ; et/ou
dans lequel la surface extérieure (14) ou au moins une section de la surface extérieure (14) s'étend le long d'un côté d'un cylindre de référence, dans lequel un axe du cylindre de référence est aligné avec la ligne de référence verticale (a), et dans lequel, éventuellement, la première surface (4) et/ou la partie inférieure (2) et/ou la partie intermédiaire (22) et/ou l'élément déflecteur (20) est/sont entièrement compris dans le cylindre de référence ; et/ou
dans lequel la flexion de la partie latérale (12) vers l'intérieur fléchit la partie latérale (12) vers la ligne de référence verticale (a) et/ou réduit l'angle intérieur (18), dans lequel, éventuellement, la force de restauration élastique agit pour fléchir la partie latérale (12) à l'écart de la ligne de référence verticale (a) et/ou pour augmenter l'angle intérieur (18).

10. Insert (10) selon l'une quelconque des revendications précédentes,
dans lequel l'élément déflecteur (20) comprend ou est composé d'un élastomère ; et/ou
dans lequel l'élément déflecteur (20) s'étend vers le haut à partir de la partie inférieure (2); et/ou
dans lequel l'élément déflecteur (20) s'étend vers l'intérieur à partir de la partie latérale (12); et/ou
dans lequel l'élément déflecteur (20) est en retrait au-dessus de la ligne de pli horizontale (c).

11. Insert (10) selon l'une quelconque des revendications précédentes, lequel comprend en outre :
une deuxième ouverture (72) adaptée pour recevoir une optode ou pour permettre une ligne de visée depuis le dessous du flacon (30) vers le volume intérieur (36) du flacon (30), en particulier, dans laquelle la deuxième ouverture (72) a une forme d'extrusion correspondant à une extrusion d'une base dans un plan horizontal dans la direction verticale ; et/ou
un évidement (74) adapté pour recevoir une pilule d'alimentation ou une cartouche d'alimentation ou une pilule tampon ou une cartouche tampon, en particulier, dans lequel l'évidement est orienté vers le haut ou vers l'intérieur, par exemple depuis la partie inférieure (2) et/ou depuis la partie intermédiaire (22) ; et/ou
une diode électroluminescente (76) montée sur l'insert (10), en particulier sur la partie inférieure (2) et/ou sur la partie intermédiaire (22).

12. Insert (10) selon l'une quelconque des revendications précédentes, dans lequel le flacon (30) est un flacon Erlenmeyer (30), en particulier un flacon Erlenmeyer à col large ou à col étroit (30), tel qu'un flacon Erlenmeyer à col étroit (30) selon la norme ISO 1773 ou un flacon Erlenmeyer à col large (30) selon la norme ISO 24450.

13. Procédé (60) d'insertion d'un insert (10) dans un flacon (30), l'insert (10) comprenant un matériau flexible et un élément déflecteur (20), le procédé comprenant le fait de :
plier (62) le matériau flexible par rapport à une ligne de pli (c) ;
aligner (64) la ligne de pli (c) parallèlement à un axe (A) du flacon (30) ;
insérer (66) l'insert plié (10) dans le flacon (30), avec la ligne de pli (c) alignée avec l'axe (A) du flacon (30) ; et
déplier (68), en dépliant le matériau flexible, l'insert (10) dans le flacon (30), de sorte qu'une première surface (4) orientée vers le bas ou vers le bas et vers l'extérieur et/ou une surface extérieure (14) de l'insert (10) vient en butée contre une surface intérieure (32, 34) du flacon (30), et que l'élément déflecteur (20) s'étend dans un volume intérieur (36) du flacon (30).

14. Procédé selon la revendication 13,
dans lequel le matériau flexible est un élastomère, dans lequel, éventuellement, l'élastomère favorise le dépliage (68) de l'insert (10) dans le flacon (30) ;
dans lequel, éventuellement, l'insert (10) est déplié par l'élastomère de sorte que la ligne de pli (c) est orientée parallèlement à une surface inférieure (34) du flacon (30) et/ou orthogonalement à l'axe (A) du flacon (30) ; et/ou
dans lequel l'insert (10) est déplié de sorte que l'insert (10) est entièrement positionné sous une partie de col du flacon (30) ; et/ou
dans lequel le procédé comprend en outre le fait de fixer l'insert (10) dans le flacon (30) et/ou d'enlever une section de l'insert (10) dans le flacon (30), par exemple par gravure ou calcination.

15. Procédé selon la revendication 13 ou 14, dans lequel l'insert (10) comprend une partie inférieure (2) comprenant la première surface (4), une partie latérale (12) comprenant la surface extérieure (14), une partie intermédiaire (22) reliant mécaniquement la partie inférieure (2) et la partie latérale (12), et un matériau flexible supplémentaire, tel qu'un élastomère supplémentaire, dans lequel le dépliage comprend le fait de :
agencer la première surface (4) afin qu'elle vienne en butée contre une surface intérieure inférieure (32) du flacon (30) ; et
agencer la surface extérieure (14) afin qu'elle vienne en butée contre une surface intérieure latérale (34) du flacon (30) ;
et, éventuellement, dans des modes de réalisation avec le premier élastomère : fournir, par le premier élastomère, une liaison par ajustement serré de la première surface (4) à la surface intérieure inférieure (32) du flacon (30) et/ou une liaison par ajustement serré de la surface extérieure (14) à la surface intérieure latérale (34) du flacon (30).
